# EUROPEAN PATENT APPLICATION

(11) **EP 0 813 863 A2**
(43) Date of publication of application: **29.12.1997**
(21) Application number: 97109287.9
(22) Date of filing: 09.06.1997
(51) Int. Cl.: A61K 9/14

(54) **A method for the preparation of stable aqueous suspensions, useful in pharmaceutical compositions**

(30) Priority: 18.06.1996 IT MI961236
(71) Applicant: Solchem Italiana S.p.A., 20090 Assago (MI) (IT)
(72) Inventor: Marcon, Giuliano, 20060 Cassino d'Alberi Mulazzano (MI) (IT); Mistretta, Paolo Antonio, 20060 Cassino d'Alberi Mulazzano (MI) (IT)
(74) Representative: Minoja, Fabrizio

(57) **Abstract**

A process for the preparation of prefixed granulometry aqueous dispersions of active principles which are water-insoluble or sparingly water-soluble at a substantially neutral pH, but which can be solubilized at acid or alkali pH, which comprises:
a) treatment of an aqueous suspension of the active ingredient with acids or bases until complete solubilization;
b) addition of a neutralizing or precipitating agent at a rate ranging from 0.05 to 0.1 litre/minute of solution to neutralize;
c) tangential microfiltration through ceramic membranes having a porosity ranging from 0.1 µm to 5 µm.

## Description

The present invention relates to a process for the preparation of prefixed granulometry aqueous dispersions of substantially water-insoluble active principles.

Particles of compounds having a poor water solubility are commonly employed in a wide range of uses: inks, paints, lubricants, pesticides, insecticides, fungicides, fertilizers, cosmetic lotions, detergents, ointments.

In many cases aqueous dispersions of fine particles are used to avoid risks such as inflammability and toxicity connected with organic solvents; often said dispersions have a very wide particle distribution.

In a number of uses, the performance of the product is increased controlling the distribution of the particle size.

Generally speaking, smaller particles of a compound will dissolve faster than the larger ones.

This aspect is remarkable, mainly in the therapeutic field: in many uses, the pharmacological activity of a compound is strictly related to the granulometric distribution, particularly when a medicament is poorly water-soluble.

A direct relationship exists between the dissolution rate and the surface area of the solid particle: the surface area increases as granulometry decreases.

The effect of the "particle size" has been proved by the higher dissolution rate and by the higher absorption efficiency observed after micronization, compared with simply grounded medicaments.

This general rule does not always apply: some very poorly soluble compounds were found to have, even after micronization, dissolution and bioavailability similar to those of the simply grounded substances themselves.

The micronization of hydrophobic powders can cause aggregation, flotation and sedimentation, when the substance is dispersed in the dissolution medium.

A plausible explanation of such an abnormal behaviour can be that these compounds have hydrophobic surfaces and therefore tend to absorb air on their surface, which inhibits their wettability.

The increase in the surface area involves a parallel increase in the absorbed air and a decrease in the surface area available to the dissolution medium, therefore the dissolution rate decreases.

This inhibitory effect can be reduced removing the absorbed air or adding small amounts of surfactants.

The processes conventionally used in the pharmaceutical industry and in other fields for the preparation of stable aqueous suspensions consist of the following steps:
1 - filtration of the active compound from the reaction mass;
2 - drying;
3 - micronization and sieving of the powder;
4 - dispersion in water with the use of specific equipments such as turbo-dispersers;
5 - addition of suspending substances;
6 - packaging of the active compound.

This general scheme evidences the high number of steps involved in the use of a specific apparatus (micronizer, turbo-dispersers) to obtain stable aqueous suspensions.

Moreover, some preparations must meet biological requirements requested by the official pharmacopoeias, thus involving the obligation to carry out periodical sanitation operations of all of the used equipments.

The subdivision of the operations, as reported above, evidently involves a remarkable care in respecting the good manufacturing practice rules envisaged by the health laws.

The procedure of the invention, which is effective mainly with compounds having a water-solubility lower than 1%, allows to obtain aqueous dispersions of compounds with prefixed granulometry according to a simplified operative scheme.

The invention therefore relates to a process for the preparation of aqueous dispersions with prefixed granulometry of active principles which are water-insoluble or sparingly water-soluble at substantially neutral pH, but can be solubilized at acid or alkali pH, which process comprises:
a) treatment of an aqueous suspension of the active ingredient with acids or bases until complete solubilization;
b) addition of a neutralizing or precipitating agent at a rate ranging from 0.05 to 0.1 litre/minute of solution to neutralize;
c) tangential microfiltration through ceramic membranes having a porosity ranging from 0.1 µm to 5 µm.

In step a), acids or bases will of course be used depending on the respectively basic or acid nature of the insoluble active ingredient. Inorganic acids or alkali hydroxides are preferably used as neutralizing agents.

Step b) is effected at a temperature ranging from - 10 to +100°C.

The particle size can suitably be controlled changing the temperature and the neutralizing agent addition rate.

The resulting suspension is then subjected to dialysis using preferably a tangential microfiltering installation fitted with a rotary pump and a ceramic membrane with a prefixed porosity.

The membranes used are made of zirconium or alumina and can operate up to 150°C: these conditions of maximum temperature also allow to carry out sanitation and/or sterilization operations particularly important for the pharmacologically active compounds.

The aqueous suspension is circulated tangentially with a high flow rate at a temperature of 0/100°C: thereby only the inorganic salt permeates.

Examples of active principles which can be advantageously used according to the present invention comprise acyclovir, oxaprozin and sucralfate. Other active principles of organic nature can anyhow be used characterized by poor or no water solubility. The technique can be applied to soluble active principles as well, even though in this case the yields will be lower due to losses in the active ingredient in the aqueous phase.

The following examples further illustrate the invention.

### Example 1

### Preparation of an aqueous suspension containing sucralfate

1420 g of Sucralfate powder are added to 120 l of a 1N hydrochloric acid aqueous solution: the suspension immediately dissolves.

The solution is decolourized through charcoal and subsequently added with 2N sodium hydroxide to pH 4.4÷4.5: a fine sulcralfate precipitate is obtained.

The resulting suspension is dialysed with a tangential microfiltering installation fitted with a 0.8 µm ceramic membrane.

During this dialysis step sodium chloride is removed to the required values.

The operation is continued with the same membrane, concentrating the aqueous suspension to a concentration of 20÷21% w/v of sucralfate in water.

The resulting suspension is ready for the final pharmaceutical formulation.

### Characteristics of the obtained suspension:

| | |
|---|---|
| Sucralfate content | 20% w/v |
| Chlorides | less than 1% on the dry substance |
| Density suspension | 1.10%÷1.50 g/ml |
| Particle size | 100% lower than 10 µm 90% comprised 1÷5 µm. |

Said suspension has a thixotropic rheological behaviour, becoming particularly fluid upon stirring.

### Example 2

### Preparation of an aqueous suspension containing acyclovir

1190 g of Acyclovir powder are added to 10 l of 0.5N sodium hydroxide; the resulting solution is filtered through charcoal, then neutralized to pH 6.7÷7.0 with 1N hydrochloric acid.

A very fine acyclovir precipitate is obtained.

The resulting suspension is dialyzed with a tangential microfiltering installation fitted with a 0.2 µm ceramic membrane.

During this dialysis step salts are removed to the required values.

The operation is continued until a 10÷12% w/v acyclovir concentration in water is obtained.

The resulting suspension is ready for the final pharmaceutical formulation.

Characteristics of the obtained suspension: particle size distribution in the range of 1÷5 µm.

### Example 3

### Stable aqueous suspension of oxaprozin calcium salt

1000 g of β(4,5-diphenyloxazol-2-yl)propionic acid are added to 10 l of distilled water, and a 50% sodium hydroxide aqueous solution is added (275 g).

The resulting sodium salt solution is added with a calcium chloride dihydrate aqueous solution (300 g): thereby the oxaprozin calcium salt precipitates in the form of a finely dispersed solid.

During this dialysis step the salts are removed.

The operation is continued concentrating the suspension to a 20-24% concentration; the resulting concentrate is added with excipients such as flavours, sweetening agents and preservatives.

The microscope analysis of the thus dispersed solid shows a distribution of the active ingredient particles in the range of 1÷10 µm.

## Claims

1. A process for the preparation of prefixed granulometry aqueous dispersions of active principles which are water-insoluble or sparingly water-soluble at substantially neutral pH, but can be solubilized at acid or alkali pH, which comprises:
a) treatment of an aqueous suspension of the active ingredient with acids or bases until complete solubilization;
b) addition of a neutralizing or precipitating agent at a rate ranging from 0.05 to 0.1 litre/minute of solution to neutralize;
c) tangential microfiltration through ceramic membranes having a porosity ranging from 0.1 µm to 5 µm.

2. A process according to claim 1 wherein the neutralizing agent is an inorganic acid.

3. A process according to claim 1 wherein the neutralizing agent is an alkali hydroxide.

4. A process according to any one of the above claims wherein step b) is effected at a controlled temperature ranging from 10 to 100°C.

5. A process according to any one of the above claims, characterized in that an aqueous dispersion of particles with a controlled, homogeneous granulometry ranging from 1 to 100 µm is obtained.

6. A process according to any one of the above claims wherein the active principles are selected from sucralfate, acyclovir and oxaprozin.
